# EUROPÄISCHE PATENTANMELDUNG

(11) **EP 1 317 936 A2**
(43) Veröffentlichungstag der Anmeldung: **11.06.2003**
(21) Anmeldenummer: 02027316.5
(22) Anmeldetag: 06.12.2002
(51) Int. Cl.: A61M 1/00

(54) **Vorrichtung zur Ausbringung von Luft aus dem Thoraxraum eines Patienten**

(30) Priorität: 06.12.2001 DE 10159960
(71) Anmelder: Heraeus Med GmbH, D-63450 Hanau (DE)
(72) Erfinder: Scholz, Bernhard, 63505 Langenselbold (DE)
(74) Vertreter: Schaumburg, Thoenes & Thurn

(57) **Zusammenfassung**

Zur Ausbringung von Luft aus dem Thoraxraum eines Patienten ist ein an eine Vakuum-Quelle anschließbares Gefäß (1) vorgesehen, dessen Innenraum eine Kontroll-Flüssigkeit aufweist, wobei der Innenraum des Gefäßes mittels eines Deckels (2) gegenüber der äußeren Atmosphäre verschließbar ist; vom Deckel (2) aus gesehen ragt ein erstes Tauchrohr (5), das mit einem patientenseitigen Schlauch in Luftverbindung steht, in den Innenraum des Gefäßes (1), weiterhin ragt vom Deckel (2) aus gesehen ein zweites, mit der Außenatmosphäre verbundenes Tauchrohr (15) in den gleichen Innenraum, wobei das Längenverhältnis der jeweils geschlossenen Rohrwandbereiche des ersten (5) und des zweiten Tauchrohres (15) kleiner als 1:1 ist.

Die Differenz der Länge von jeweils geschlossenen Rohrwandbereichen des ersten und zweiten Tauchrohres (5, 15) liegt im Bereich von 100 mm bis 500 mm. Das zweite Tauchrohr (15) ist von einem im unteren Bereich geöffneten Hüllrohr (17) umgeben, das in seinem oberen Bereich seitliche Öffnungen (18) aufweist, um den Zutritt eines Wasser-Luftgemisches zur Vakuumquelle zu verhindern.

Als besonders vorteilhaft erweist es sich, dass das am Patienten angelegte Vakuum genau gemessen und eingestellt werden kann, wobei die Vorrichtung einen kompakten Aufbau aufweist, so dass ein Patient sich wenigstens im beschränkten Rahmen auch ohne ortsfeste Verbindung bewegen kann. Die Vorrichtung ist einfach einzurichten, mehrfach verwendbar und vereint alle für eine Thoraxdrainage notwendigen Mess- und Sicherheitseinrichtungen in sich.

## Beschreibung

Die Erfindung betrifft eine Vorrichtung zur Ausbringung von Luft aus dem Thoraxraum eines Patienten mit einem an eine Vakuumquelle anschließbaren Gefäß, dessen Innenraum zur Aufnahme einer Kontroll-Flüssigkeit vorgesehen ist, wobei der Innenraum des Gefäßes mittels eines Deckels gegenüber der äußeren Atmosphäre verschließbar ist und vom Deckel ein erstes Tauchrohr, das mit einem patientenseitigen Schlauch in Luftverbindung steht, in den Innenraum des Gefäßes ragt.

Als Tauchrohr wird ein in die Kontrollflüssigkeit teilweise getauchtes Rohr bezeichnet, dessen unteres Ende offen ist.

Aus der DE 200 11 287 U1 ist eine Auffangeinrichtung für pathologische Flüssigkeiten und von Luft aus dem Thoraxraum eines Patienten im Wege einer Drainage unter Ausnützung des hydrostatischen Druckgefälles bzw. durch Absaugen der Flüssigkeit mit Unterdruck bekannt, wobei ein Schlauch mit einem Katheteranschlussstück in einen Behälter hinein zu einem in die Kontrollfiüssigkeit tauchenden Röhrchen führt und einen Behälterverschluss mit einer Öffnung zum Austritt von Luft aufweist. In dem ggf. abnehmbaren Deckel des Behälters ist ein verschließbarer Anschluss zur sterilen Befüllung des Behälters mit einer Kontrollflüssigkeit wie NaCI und ein Ausströmventil für Luft versehen, welches beispielsweise als Schirmventil mit Membran vorgesehen ist.

Als problematisch erweist es sich, dass eine möglichst exakte Einstellung des patientenseitigen Vakuums nicht möglich ist, weil das Maß, um das das Röhrchen in die Kontrollflüssigkeit getaucht wird, nicht bestimmt ist und sich bei anfallendem Sekret auch noch verändert. Das am Thorax wirkende Vakuum wird durch den sich ggf. ändernden hydrostatischen Druck verringert, was grundsätzlich zu Messfehlern des wirksamen patientenseitigen Vakuums führt, und unter Umständen sogar das Vakuum am Thorax vollständig zusammenbrechen lässt.

Bei derzeit handelsüblichen Vorrichtungen zur Ausbringung von Luft aus dem Thoraxraum eines Patienten ist es als problematisch anzusehen, dass bei einer starken Vakuumerhöhung ein Wasserluftblasengemisch entsteht, das zu einem Ansteigen der Wassersäule führt, wobei im ungünstigen Fall auch Flüssigkeit von der Vakuumquelle angesaugt wird; weiterhin muss mit einer Verdunstung durch Blasenbildung gerechnet werden, da eine solche Vorrichtung in der Regel für mehrere Tage am Patienten wirksam ist und das Vakuum als eingestellt gilt, wenn Luftblasen aus dem mit der Atmosphäre verbundenen Tauchrohr steigen.

Aufgabe der Erfindung ist es, eine Integration von bisher in der Praxis häufig voneinander getrennten Sicherheitseinrichtungen für die Thoraxdrainage in einer kompakten Vorrichtung zusammenzufassen, woraus sich eine Vereinfachung von Thorax-Drainagen für das Krankenhauspersonal hinsichtlich Einrichtung und Gebrauch ergeben soll.

Weiterhin soll das am Patienten angelegte Vakuum genauer gemessen werden und eine eventuelle Änderung des Wertes der Vakuumeinstellung vermieden werden.

Die Aufgabe wird dadurch gelöst, dass vom Deckel ausgehend ein zweites, mit der Außenatmosphäre verbundenes Tauchrohr in den gleichen Innenraum des Gefäßes ragt, wobei das Längenverhältnis der jeweils geschlossenen Rohrwandbereiche des ersten und des zweiten Tauchrohres kleiner als 1:1 ist.

Als besonders vorteilhaft erweist es sich, dass das Gerät über eine Steckverbindung direkt an eine Vakuumquelle angeschlossen werden kann, bzw. dass an den Vakuumanschluss ein "Vakuumbehälter" angeschlossen wird, der es dem Thoraxpatienten erlaubt, über längere Zeit mobil zu bleiben, weil das am Thorax angelegte Vakuum aufrecht erhalten bleiben kann.

Vorteilhafte Ausgestaltungen der Erfindung nach Anspruch 1 sind in den Ansprüchen 2 bis 8 angegeben.

In einer bevorzugten Ausgestaltung der Erfindung liegt die Differenz der Länge von jeweils geschlossenen Rohrwandbereichen des ersten und zweiten Tauchrohres im Bereich von 100 mm bis 500 mm, vorzugsweise im Bereich von 200 bis 300 mm. Somit ragt das mit der Atmosphäre verbundene zweite Tauchrohr tiefer in die Flüssigkeit ein als das mit dem patientenseitigen Schlauch verbundene erste Tauchrohr.

Vorzugsweise ist das mit der Atmosphäre verbundene zweite Tauchrohr von einem längeren und an seinem unteren Ende geöffneten Hüllrohr umgeben, wobei das Hüllrohr zwischen dem zur Aufnahme der Flüssigkeit vorgesehenen Bereich (Flüssigkeitsspiegel) und dem Deckel wenigstens eine seitliche Öffnung in seiner Rohrwand aufweist, so dass Luftblasen aus einem eventuellen Luft-Wasser-Gemisch ohne Wasserpartikel zur Vakuumquelle abgesaugt werden können. Das Hüllrohr oder das mit der Atmosphäre verbundene zweite Tauchrohr weist dabei eine Skala als Maß für den Unterdruck im Innenraum des Gefäßes auf.

In einer vorteilhaften Ausgestaltung weist das mit der Atmosphäre verbundene zweite Tauchrohr einen Schwimmkörper zur optischen Anzeige des Unterdrucks auf.
Zur verbesserten Beobachtung besteht wenigstens ein Teil der Seitenwand des Behälters aus optisch transparentem Werkstoff, wobei auch die beiden Tauchrohre - zumindest das zweite Tauchrohr - und das Hüllrohr wenigstens zum Teil aus optisch transparentem Werkstoff bestehen.

Als vorteilhaft erweist es sich, dass das am Patienten angelegte Vakuum genau gemessen und eingestellt werden kann, wobei die Vorrichtung einen kompakten Aufbau aufweist, so dass ein Patient sich wenigstens in einem beschränkten Bereich auch ohne ortsfeste Verbindung frei bewegen kann. Die Vorrichtung ist einfach einzurichten, mehrfach verwendbar und vereint alle für eine Thoraxdrainage notwendigen Mess- und Sicherheitseinrichtungen in sich.

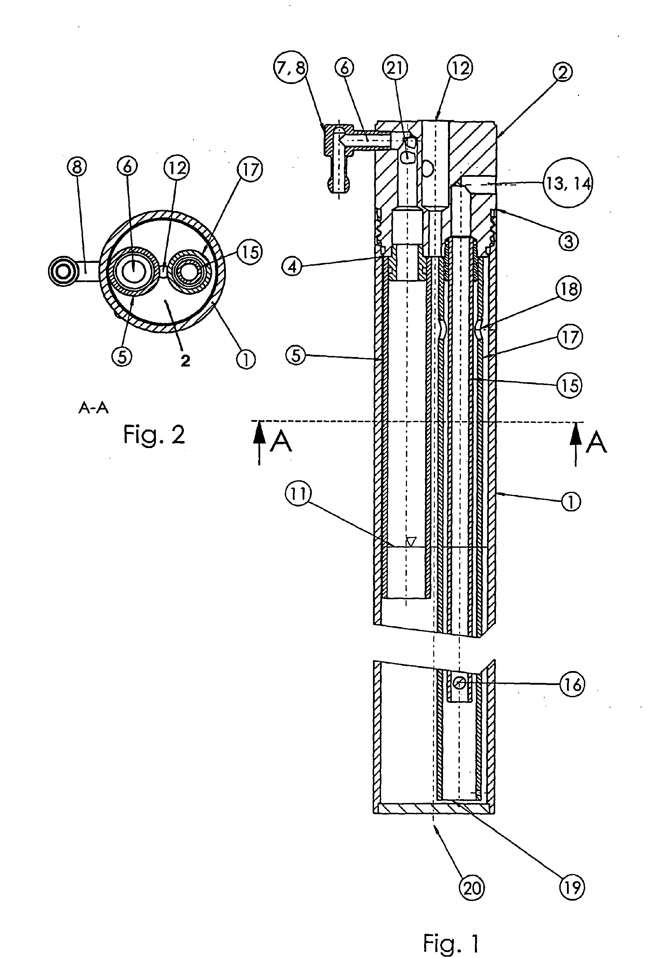

Im folgenden ist der Gegenstand der Erfindung anhand der Figuren 1 und 2 näher erläutert.

Figur 1 zeigt die erfindungsgemäße Vorrichtung im Längsschnitt, wobei sich sämtliche Anschlüsse im Deckelbereich eines Gefäßes befinden, wobei auch die entsprechenden Tauchrohre im Längsschnitt dargestellt sind.

Figur 2 zeigt einen Querschnitt entlang der mit AA bezeichneten Fläche der Vorrichtung nach Figur 1, wobei die Tauchrohre sowie der Deckel erkennbar sind.

Gemäß Figur 1 weist die Vorrichtung ein Gefäß 1 zur Aufnahme von Flüssigkeit auf, welches vorzugsweise als hohlzylindrisches Gefäß aus transparentem Werkstoff ausgebildet ist. An seiner Oberkante ist das Gefäß mit einem Deckel 2 verschlossen, welcher den oberen Rand 3 des Gefäßes gegenüber der Außenatmosphäre abschließt. Der Deckel 2 weist an seiner in das Gefäß 1 ragenden Unterseite 4 ein vertikal nach unten gerichtetes erstes Tauchrohr 5 auf, welches an seiner Unterkante geöffnet ist und im Bereich des Deckels 2 mit einem (schematisch dargestellten) Luftkanal 6 zu einem Anschluss 8 für einen patientenseitigen Schlauch 7 versehen ist.

Zwischen Anschluss 8 und dem patientenseitigen Katheter ist ein hier nicht gezeigtes Gefäß zur Sekretabscheidung vorgesehen.

Das Tauchrohr 5 weist mit seiner geschlossenen Rohrwand eine Länge auf, die bis unter einen für Inbetriebnahme der Vorrichtung vorgegebenen Wasserspiegel 11 reicht. Das Tauchrohr dient dabei als "Wasserschloss" oder "Rückschlag-Ventil", falls die Vakuumquelle - z.B. infolge eines Fehlers in der Stromversorgung - ausfallen sollte. In einem solchen Fall wird der patientenseitige Unterdruck wenigstens für eine kurze Zeit aufrechterhalten, so dass Fachpersonal oder ggf. der Patient selbst die erforderlichen Umschaltmaßnahmen zum weiteren Betrieb der Vakuumquelle vornehmen kann. Die optimale Eintauchtiefe des ersten Tauchrohres liegt im Bereich von 20 bis 50 mm.

Weiterhin weist der Deckel 2 einen Kanal 12 zu einer hier nicht gezeigten Vakuumquelle auf, welche zur Erzeugung eines Unterdrucks im Innenraum von Gefäß 1 dient.

Darüber hinaus ist im Bereich von Anschluss 8 ein Überdruckventil 21 angedeutet, wodurch ein eventueller Überdruck auf der Patientenseite - wie er z.B. beim Husten auftreten kann - nach außen zur Atmosphäre abgeführt wird. Das Überdruckventil 21 ist vorteilhafterweise in Form eines Membranventils ausgeführt und kann an Kanal 6 oder 12 angeschlossen sein.

Der Deckel 2 ist weiterhin mit einem Kanal 14 zur Luftverbindung mit der Außenatmosphäre versehen, welcher mit einem in das Innere von Gefäß 1 ragenden zweiten Tauchrohr 15 nahezu bis in den Bodenbereich von Gefäß 1 reicht: es ist an seiner Unterseite ebenfalls geöffnet und enthält im Inneren einen Schwimmkörper 16, um einen Messvorgang zur Einstellung für das patientenseitige Vakuum zu erleichtern. Das zweite Tauchrohr 15 ist seinerseits von einem Hüllrohr 17 umgeben, welches wenigstens teilweise als Tauchrohr ausgebildet ist und im unteren Bereich 20 des Gefäßes 1 eine Öffnung 19 aufweist. Das Hüllrohr 17 sorgt dafür, dass eventuell über das zweite Tauchrohr von außen einströmende Luft direkt in den oberen Bereich des Gefäßes 1 geführt wird, wobei es durch seitliche Öffnungen 18 austritt, die im Abstand oberhalb des vorgegebenen Wasserspiegels 11 liegen, so dass kein Wasser-Luftgemisch mehr zur Vakuumquelle gelangen kann.

Gemäß Figur 2 ist das erste Tauchrohr 5 im Querschnitt erkennbar, wobei auch die in Deckel 2 eingebrachte Öffnung des Kanals 6 zum patientenseitigen Anschluss 8 erkennbar ist; die Weiterführung des im Querschnitt an sich nicht erkennbaren Kanals ist durch eine gestrichelte Linie angedeutet, wobei der Anschluss 8 für den patientenseitigen Schlauch wiederum von seiner Unterseite sichtbar ist.

Weiterhin ist im Zentrum entlang der Achse 20 des Gefäßes 1 (Figur 1) der Kanal 12 zur Vakuumquelle erkennbar, welcher in der Praxis durch eine Schlauch- oder Steckverbindung mit einer hier nicht dargestellten Vakuumpumpe verbunden ist.

In der rechten Hälfte von Figur 2 ist das zweite Tauchrohr 15 als Messrohr sowie Hüllrohr 17 erkennbar, welches über einen hier nicht sichtbaren - jedoch in Figur 1 gestrichelt dargestellten Luftkanal 14 - zur Außenatmosphäre geführt ist, wobei der Anschluss zur Außenatmosphäre über einen hier nicht dargestellten Schlauch mit Anschluss 13 (Figur 1) und ggf. vorgeschaltetem Filter bzw. Luftreinigung erfolgen kann. Es ist jedoch auch möglich, Anschluss 13 lediglich als Öffnung zur Außenatmosphäre vorzusehen.

## Patentansprüche

1. Vorrichtung zur Ausbringung von Luft aus dem Thoraxraum eines Patienten mit einem an eine Vakuum-Quelle anschließbaren Gefäß (1), dessen Innenraum zur Aufnahme einer Kontroll-Flüssigkeit vorgesehen ist, wobei der Innenraum des Gefäßes (1) mittels eines Deckels (2) gegenüber der äußeren Atmosphäre verschließbar ist und vom Deckel (2) ein erstes Tauchrohr (5), das mit einem patientenseitigen Schlauch in Luftverbindung steht, in den Innenraum des Gefäßes (1) ragt, **dadurch gekennzeichnet, daß** vom Deckel (2) ausgehend ein zweites, mit der Außenatmosphäre verbundenes Tauchrohr (15) in den gleichen Innenraum ragt, wobei das Längenverhältnis der jeweils geschlossenen Rohrwandbereiche des ersten (5) und des zweiten Tauchrohres (15) kleiner als 1:1 ist.

2. Vorrichtung nach Anspruch 1, **dadurch gekennzeichnet, daß** die Differenz der Länge von jeweils geschlossenen Rohrwandbereichen des ersten und zweiten Tauchrohres (5, 15) im Bereich von 100 mm bis 500 mm liegt.

3. Vorrichtung nach Anspruch 1 oder 2, **dadurch gekennzeichnet, daß** das mit der Atmosphäre verbundene zweite Tauchrohr (15) von einem etwas längeren Hüllrohr (17) umgeben ist, wobei das Hüllrohr (17) zwischen dem zur Aufnahme der Flüssigkeit vorgesehenen Bereich und dem Deckel (2) wenigstens eine seitliche Öffnung (18) in seiner Rohrwand aufweist.

4. Vorrichtung nach Anspruch 3, **dadurch gekennzeichnet, daß** das Hüllrohr (17) oder das mit der Atmosphäre verbundene zweite Tauchrohr (15) eine Skala als Maß für den Unterdruck im Innenraum des Gefäßes (1) aufweist.

5. Vorrichtung nach einem der Ansprüche 1 bis 4, **dadurch gekennzeichnet, daß** das mit der Atmosphäre verbundene Tauchrohr (15) einen Schwimmkörper (16) zur optischen Anzeige des Unterdrucks aufweist.

6. Vorrichtung nach einem der Ansprüche 1 bis 5, **dadurch gekennzeichnet, daß** wenigstens ein Teil der Seitenwand des Gefäßes (1) aus optisch transparentem Werkstoff besteht.

7. Vorrichtung nach einem der Ansprüche 3 bis 6, **dadurch gekennzeichnet, daß** das Hüllrohr (17) und das zweite Tauchrohr (15) wenigstens zum Teil aus optisch transparentem Werkstoff bestehen.
